# EUROPEAN PATENT SPECIFICATION

(11) **EP 2 633 809 B1**
(45) Date of publication and mention of the grant of the patent: **05.07.2017**
(21) Application number: 13156402.3
(22) Date of filing: 22.02.2013
(51) Int. Cl.: A61B 5/044

(54) **Electrocardiogram analysis apparatus, and electrocardiogram analysis program**
Elektrokardiogramm-Analysevorrichtung und Elektrokardiogramm-Analyseprogramm
Appareil et programme d'analyse d'électrocardiogramme

(30) Priority: 02.03.2012 JP 2012046831; 06.11.2012 JP 2012244754
(43) Date of publication of application: 04.09.2013
(73) Proprietor: Nihon Kohden Corporation, Shinjuku-ku Tokyo 161-8560 (JP)
(72) Inventor: Takayanagi, Tsuneo, Tokyo, 161-8560 (JP); Suto, Jiro, Tokyo, 161-8560 (JP); Kamiya, Ryoko, Tokyo, 161-8560 (JP); Ukawa, Teiji, Tokyo, 161-8560 (JP); Hagiwara, Hiroko, Tokyo, 161-8560 (JP); Muneshima, Rie, Tokyo, 161-8560 (JP)
(74) Representative: McCartney, Jonathan William

(56) References cited:
- WO-A1-2011/121494
- WO-A2-2006/033038
- DE-A1-102010 060 752
- JP-A- H0 970 395
- US-A1- 2011 060 234

## Description

### BACKGROUND

This invention relates to an electrocardiogram analysis apparatus, and an electrocardiogram analysis program.

A method of outputting an electrocardiogram analysis result with easy understanding is being required. The electrocardiogram analysis is generally summarized in an electrocardiogram analysis report prior to output (for example, displayed on a display or printed on a paper). The electrocardiogram analysis report includes an electrocardiogram waveform and a variety of information such as an ST level acquired from the electrocardiogram waveform, and a medical staff such as a doctor determines a heart state of a subject with reference to the electrocardiogram analysis report.

Therefore, it is very important to output an electrocardiogram analysis report such that a medical staff can easily understand the electrocardiogram analysis report in determining a heart state of a subject accurately and rapidly. For example, Japanese Patent No. 3040701 discloses an electrocardiogram information output method that outputs an electrocardiogram waveform and an ST level together with the illustration of a human body in the order corresponding to an angle of a lead axis.

However, in the electrocardiogram information output method disclosed in Japanese Patent No. 3040701, since an ST level is output in a bar graph, it is difficult to know in which part of a heart an electrocardiogram trouble such as an ST increase has occurred. Also, in a horizontal plane (chest lead), an electrocardiogram waveform of a chest lead is arranged in the direction from the head side to the heart side. However, in a recently-popularized diagnostic imaging apparatus such as CT or MRI, an image is created in the direction from the foot side to the heart side. When both cases are considered, since viewing directions are different even on the same plane, it is difficult to know the correspondence relation between the arrangement of an electrocardiogram waveform and the arrangement of a part of a heart in a diagnostic imaging apparatus.

WO 2006/033038 A2 discloses an electrocardiogram analysis apparatus comprising the features of the preamble of claim 1.

This invention has been made to address the above-described problem. Therefore, an object of this invention is to provide an electrocardiogram analysis report that makes it easy to know in which part of a heart an electrocardiogram trouble such as an ST increase has occurred. Another object of this invention is to provide an electrocardiogram analysis report that makes it easy to know the correspondence relation between the arrangement of an electrocardiogram waveform and the arrangement of a part of a heart in a diagnostic imaging apparatus.

### SUMMARY

In order to achieve the above object, an electrocardiogram analysis apparatus as defined in claim 1, and a computer-readable recording medium storing an electrocardiogram analysis program as defined in claim 9 are provided. Preferred embodiments are defined in the dependent claims. For example, the display of the measurement value is changed with a different mark according to whether the measurement value is positive or negative, thereby making it possible to immediately recognize the positive/negative information of the measurement value. Also, a normal range and an alarm range are displayed on the same plot, thereby making it possible to immediately recognize at which part the measurement value has deviated from the normal range, or whether the measurement value has deviated from the alarm range. In addition, a numerical value of the measurement value is displayed, thereby making it possible to recognize a numerical value of the measurement value having a trouble on the plot and thus to make a more detailed determination.

Also, a corresponding part of a heart is displayed in characters on the electrocardiogram analysis report, thereby making it easy to recognize the relation between the electrocardiogram or the measurement value and the part of a heart.

Also, electrocardiogram waveforms and information about measurement values at different times are simultaneously displayed, thereby making it possible to comparatively evaluate how much the measurement value of any part has changed at different times, or whether the measurement value of any part has not changed. In addition, with respect to the waveform and the measurement value, the electrocardiogram waveforms at different times are displayed in a superimposed manner, and a changed ST portion is painted in a specific color, thereby making it easier to recognize a measurement value change.

Also, in addition to a standard 12-lead, an additional chest lead (including waveforms combined from a 12-lead electrocardiogram, such as V3R, V4R, V5R, V7, V8, and V9) is together displayed, thereby making it possible to recognize an phenomenon occurring in the heart by using a wider range of information.

In addition, an upper limit value and a lower limit value of an alarm about the measurement value are displayed, thereby specifying that the measurement value has deviated from a range of the upper limit value or the lower limit value of the alarm. The electrocardiogram analysis apparatus includes an analysis part configured to analyze an electrocardiogram waveform collected from a subject, and an output part configured to output an electrocardiogram analysis report based on an analysis result of the electrocardiogram waveform. The analysis part calculates a measurement value based on each of a limb leads waveform and a chest lead waveform included in the electrocardiogram waveform. The output part creates the electrocardiogram analysis report and outputs the same on a display or a paper. The output part includes a frontal plane display unit and a horizontal plane display unit. The frontal plane display unit outputs the limb leads waveform and information about the measurement value. The horizontal plane display unit outputs the chest lead waveform and information about the measurement value. The frontal plane display unit and the horizontal plane display unit output the information about the measurement value at a position on an output surface spaced apart by a distance corresponding to a size of the measurement value in a direction from a predetermined center point to the relevant lead waveform.

In addition, subject electrocardiogram information varying from hour to hour is displayed in real time, thereby making it possible to recognize the latest information of the subject. Also, information about an electrocardiogram waveform and a measurement value is continuously displayed, thereby making it possible to visually easily recognize when and what types of troubles have occurred.

In addition, with respect to the waveform and the measurement value, the electrocardiogram waveforms at different times are displayed in a superimposed manner, and a changed ST portion is painted in a specific color, thereby making it easier to recognize a measurement value change.

In addition, an upper limit value and a lower limit value of an alarm about the measurement value are output, thereby specifying that the measurement value has deviated from a range of the upper limit value or the lower limit value of the alarm. The electrocardiogram analysis program includes an analysis function of analyzing an electrocardiogram waveform collected from a subject, and an output function of outputting an electrocardiogram analysis report based on an analysis result of the electrocardiogram waveform. The analysis function calculates a measurement value based on each of a limb leads waveform and a chest lead waveform included in the electrocardiogram waveform. The output function creates the electrocardiogram analysis report and outputs the same on a display or a paper. The output function includes a frontal plane display function and a horizontal plane display function. The frontal plane display function outputs the limb leads waveform and information about the measurement value. The horizontal plane display function outputs the chest lead waveform and information about the measurement value. The frontal plane display function and the horizontal plane display function output the information about the measurement value at a position on an output surface spaced apart by a distance corresponding to a size of the measurement value in a direction from a predetermined center point to the relevant lead waveform.

In addition, subject electrocardiogram information varying from hour to hour is displayed in real time, thereby making it possible to recognize the latest information of the subject. Also, information about an electrocardiogram waveform and a measurement value is continuously displayed, thereby making it possible to visually easily recognize when and what types of troubles have occurred.

In addition, with respect to the waveform and the measurement value, the electrocardiogram waveforms at different times are displayed in a superimposed manner, and a changed ST portion is painted in a specific color, thereby making it easier to recognize a measurement value change.

In addition, an upper limit value and a lower limit value of an alarm about the measurement value are output, thereby specifying that the measurement value has deviated from a range of the upper limit value or the lower limit value of the alarm.

The objects, features, and characteristics of this invention other than those set forth above will become apparent from the description given herein below with reference to preferred embodiments illustrated in the accompanying drawings.

### BRIEF DESCRIPTION OF THE DRAWINGS

Fig. 1 is a block diagram illustrating a schematic configuration of an electrocardiogram analysis apparatus according to an embodiment of this invention.
Fig. 2 is a flow chart for describing a method of outputting an electrocardiogram analysis report according to an embodiment of this invention.
Fig. 3 is a diagram illustrating an example of an electrocardiogram waveform according to an embodiment of this invention.
Fig. 4 is a diagram illustrating an example of an electrocardiogram analysis report according to an embodiment of this invention.
Fig. 5 is a block diagram illustrating a schematic configuration of a biological information monitor according to another embodiment of this invention.
Fig. 6 is a diagram illustrating an example of an electrocardiogram analysis report displayed on a biological information monitor according to another embodiment of this invention.
Fig. 7 is a diagram for describing an electrocardiogram waveform comparison function according to another embodiment of this invention.
Fig. 8 is a diagram for describing an alarm range display function according to another embodiment of this invention.

### DETAILED DESCRIPTION

Hereinafter, embodiments of an electrocardiogram analysis apparatus will be described with reference to the accompanying drawings. In addition, throughout this specification, displaying on a display and printing on a paper will all be denoted as 'outputting'.

### (Embodiments)

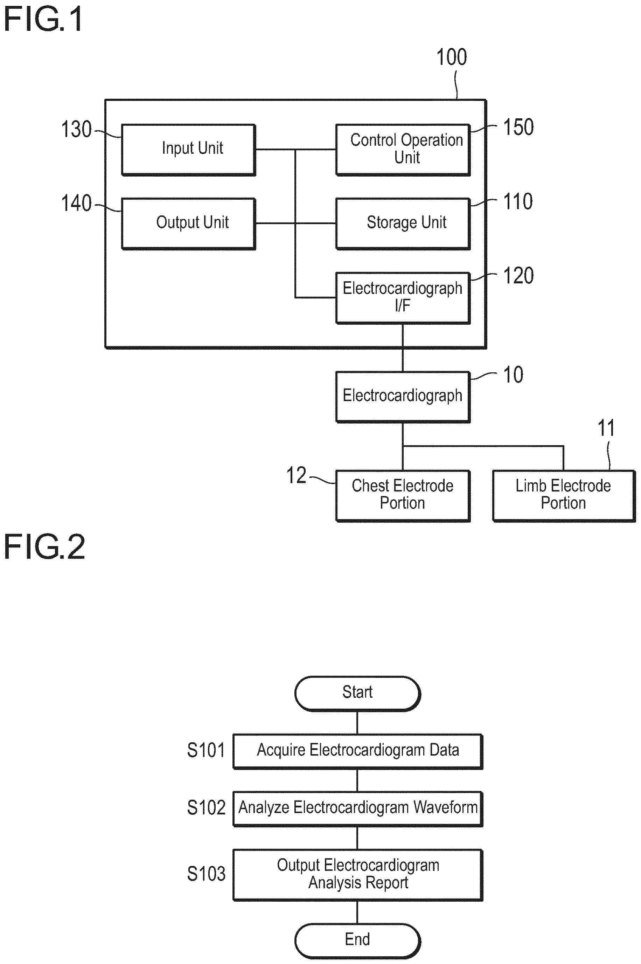

Fig. 1 is a block diagram illustrating a schematic configuration of an electrocardiogram analysis apparatus according to an embodiment. As illustrated in Fig. 1, an electrocardiogram analysis apparatus 100 according to this embodiment includes a storage unit 110, an electrocardiograph interface (hereinafter, referred to as electrocardiograph I/F) 120, an input unit 130, an output unit 140, and a control operation unit 150, and these elements are communicatively connected to each other.

The storage unit 110 stores various software programs including an electrocardiogram analysis program, various parameters, an operation result by the control operation unit 150, an electrocardiogram waveform, various measurement values, and image data of an electrocardiogram analysis report generated, and the like. The storage unit 110 uses a storage device such as a hard disk or a semiconductor memory. The electrocardiogram analysis report will be described below.

The electrocardiograph I/F 120 communicatively connects an electrocardiograph 10 and the control operation unit 150. The electrocardiograph I/F 10 acquires an electrical signal from a heart though a limb electrode portion 11 and a chest electrode portion 12, converts the electrical data into electrocardiogram data, and provides the electrocardiogram data to the electrocardiograph I/F 120. The limb electrode portion 11 includes a plurality of limb electrodes, and the chest electrode portion 12 includes a plurality of chest electrodes. The limb electrodes and the chest electrodes are attached to predetermined positions on a body surface of a subject. Also, in this embodiment, an electrocardiograph capable of collecting leads including limb leads and a chest lead may be used as the electrocardiograph 10. Hereinafter, the case of using an electrocardiograph using a standard 12-lead electrocardiogram and a 6-lead derived from a waveform thereof (hereinafter, referred to as derived 18-lead) will be described as an example. An arrangement of the lead in the chest lead corresponds to aposition of the chest electrode, when viewed from the lower side of a human body. In addition, since a technical content such as an operating principle of the electrocardiograph 10 is well known, a detailed description thereof will be omitted.

The input unit 130 receives an instruction from a medical staff. The input unit 130 includes, for example, a button, a switch, a touch panel, a keyboard, and a mouse, and transmits an instruction from a medical staff to the control operation unit 150.

The output unit 140 outputs an electrocardiogram analysis report. In this embodiment, the output unit 140 includes a display and a printer. The output unit 140 outputs an electrocardiogram analysis report received from the control operation unit 150 on a display surface as an output surface of the display, or outputs the electrocardiogram analysis report on a paper as an output surface of the printer. Alternatively, the output unit 140 may output the electrocardiogram analysis report on the display and the paper simultaneously. In addition, the output unit 140 may include any one of the display and the printer.

The control operation unit 150 includes a CPU (Central Processing Unit), and executes the various software programs and various operations. In this embodiment, the control operation unit 150 executes the electrocardiogram analysis program to acquire electrocardiogram data, analyze an electrocardiogram waveform, and generate image data of an electrocardiogram analysis report. The control operation unit 150 transmits the image data of the electrocardiogram analysis report, and stores the image data of the electrocardiogram analysis report in the storage unit 110. In this embodiment, the control operation unit 150 functions as analysis part. Also, the output unit 140 and the control operation unit 150 functions as output part.

Also, the control operation unit 150 may receive electrocardiogram data from the electrocardiograph 10 over a predetermined period and store the electrocardiogram data in the storage unit 110. The control operation unit 150 calculates a measurement value based on an electrocardiogram waveform at a predetermined time in the predetermined period and stores the measurement value in the storage unit 110. Therefore, with respect to information about an electrocardiogram waveform or a measurement value, the output unit 140 and the control operation unit 150 can simultaneously output the information that is collected or calculated at different times. As a result, it is possible to comparatively evaluate how much the measurement value of any part of the heart has changed, or whether the measurement value of any part of the heart has not changed.

Also, the control operation unit 150 continuously receives an electrocardiogram wave from the electrocardiograph 10. Therefore, the output unit 140 and the control operation unit 150 can update and output the electrocardiogram waveform and the measurement value by using the latest information about the electrocardiogram waveform and the measurement value. Also, the output unit 140 and the control operation unit 150 can continuously output the different-time information about the electrocardiogram waveform and the measurement value. Therefore, it can be easy to visually recognize when and what types of troubles have occurred in the heart.

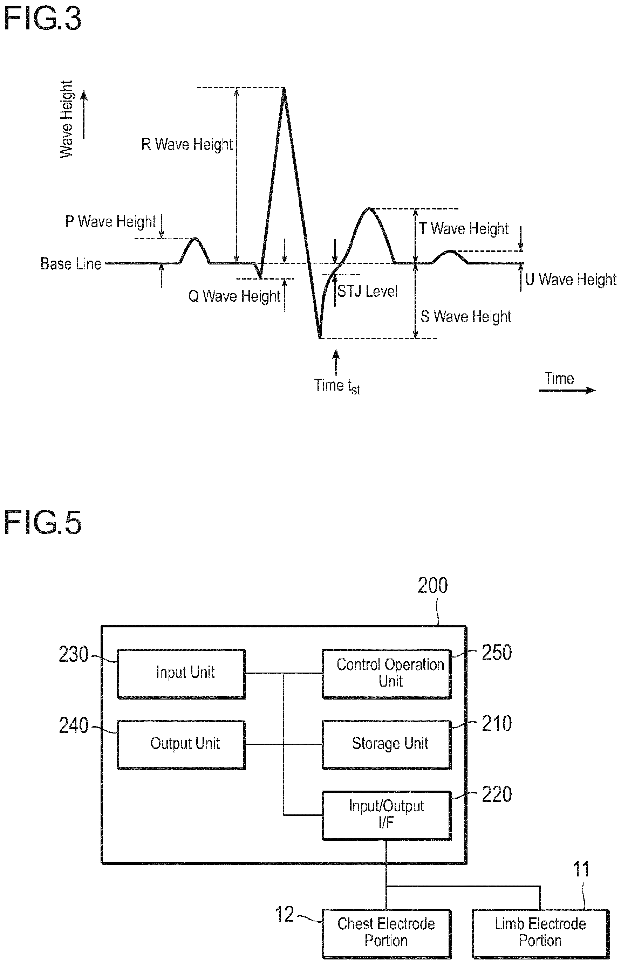

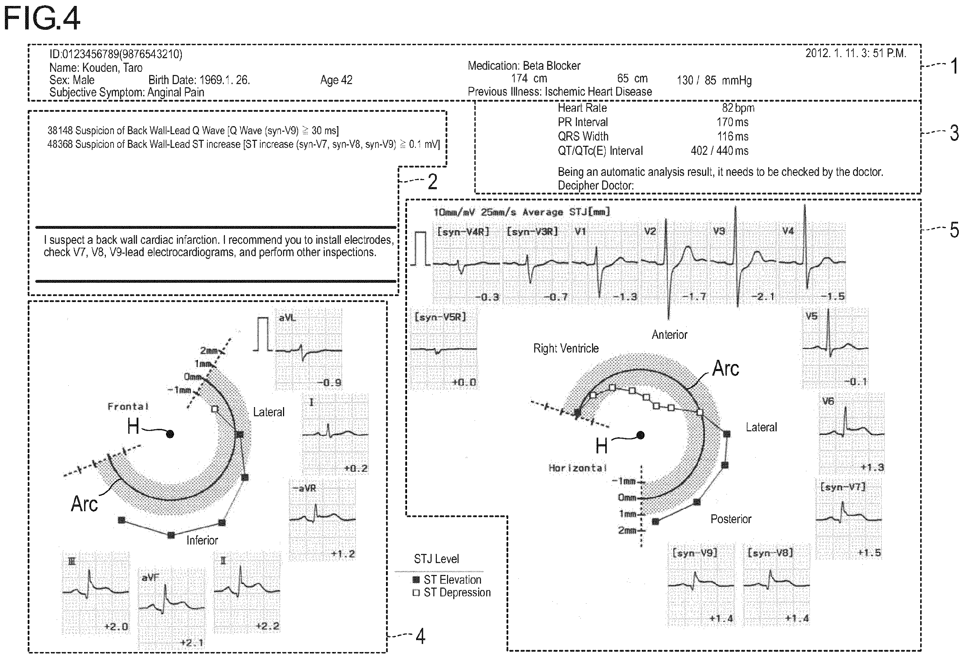

Hereinafter, a method of outputting an electrocardiogram analysis report according to this embodiment will be descried with reference to Figs. 2 to 4. Fig. 2 is a flow chart for describing a method of outputting an electrocardiogram analysis report according to this embodiment, and Fig. 3 is a diagram illustrating an example of an electrocardiogram waveform according to this embodiment. In Fig. 3, a vertical axis represents a wave height, and a horizontal axis represents a time. Also, Fig. 4 is a diagram illustrating an example of an electrocardiogram analysis report according to this embodiment.

First, as illustrated in Fig. 2, electrocardiogram data is acquired (step S101). A medical staff instructs the control operation unit 150 to receive electrocardiogram data through the input unit 130. The control operation unit 150 receives electrocardiogram datafromthe electrocardiograph 10 through the electrocardiogram I/F 120. In this embodiment, since a derived 18-lead electrocardiograph is used, the electrocardiogram data includes a lead waveform, when viewing a heart from 18 points of view.

In this embodiment, the lead includes as I lead, II lead, III lead, aVL lead, -aVR lead, and aVF lead as limb leads. Also, the lead includes syn-V5R lead, syn-V4R lead, syn-V3R lead, V1 lead, V2 lead, V3 lead, V4 lead, V5 lead, V6-lead, syn-V7 lead, syn-V8 lead, and syn-V9 as chest leads. Also, the syn-V3R lead, the syn-V4R lead, the syn-V5R lead, the syn-V7 lead, the syn-V8 lead, and the syn-V9 lead are leads derived from operation.

Next, an electrocardiogram waveform is analyzed (step S102). As illustrated in Fig. 3, the control operation unit 150 averages N-beat electrocardiogram waveforms with respect to each lead, and calculates an ST level as a measurement value from the averaged electrocardiogram waveform. Herein, N is, for example, 10, and the ST level is a difference value between a wave height and a base line at a predetermined time tst.

In this embodiment, a difference value between a wave height of an end point of a QRS and a base line (which referred to as STJ level in this specification) is used as the ST level. Hereinafter, the case of outputting the STJ level in the electrocardiogram analysis report in addition to the electrocardiogram waveform will be described. However, in the electrocardiogram analysis report, the wave heights or widths of a P wave, a Q wave, an R wave, an S wave, a T wave, and a QRS wave, a PR interval, a QT interval, and a QTc interval may also be output as measurement values, if necessary. The wave heights may be calculated as the difference value between a wave height and a base line by the control operation unit 150.

Next, an electrocardiogram analysis report is output (step S103). The control operation unit 150 outputs amessage on the display of the output unit 140 in order to urge the medical staff to input with respect to an electrocardiogram analysis report output method. According to the message, the medical staff inputs the intent to output the electrocardiogram analysis report on any one or both of a display surface of the display and a paper.

As illustrated in Fig. 4, in this embodiment, the electrocardiogram analysis report simultaneously outputs an electrocardiogram waveform and information about a measurement value calculated from the electrocardiogram waveform. The electrocardiogram analysis report includes five fields (a first field 1 to a fifth field 5) in one page. Each of the fields will be described below.

The first field 1 is a field for outputting basic data of the subject. The output data may include an ID, a name, a sex, a birth date, an age, a subjective symptom, the name of an administration medicine, a height, a weight, a blood pressure, a previous illness, and the like.

The second field 2 is a field for outputting an analysis opinion about the electrocardiogram waveform. The output data may include an automatic analysis opinion, words indicating which treatment to be performed next, and the like.

The third field 3 is a field for outputting a representative measurement value of the electrocardiogram waveform. The output data may include a heart rate, a PR interval, a QRS width, a QT interval, a QTc interval, a remark, and the like. Also, the heart rate, the PR interval, the QRS width, the QT interval, and the QTc interval may be calculated from the electrocardiogram waveform by the control operation unit 150.

The fourth field 4 is a frontal plane display region, and is a field for outputting a limb leads STJ level and an electrocardiogram waveform.

The fifth field 5 is a horizontal plane display region, and is a field for outputting a chest lead STJ level and an electrocardiogram waveform.

The control operation unit 150 calculates a position on an output surface on which each item of the first field 1 to the fifth field 5 of the electrocardiogram analysis report to be output, and generates image data of the electrocardiogram analysis report. The generated image data of an electrocardiogram analysis report is transmitted to the output unit 140 and is stored in the storage unit 110. Also, the image data of the electrocardiogram analysis report stored in the storage unit 110 may be transmitted to an external display, printer or the like through a communication interface (not illustrated).

The electrocardiogram analysis apparatus 100 according to this embodiment outputs the STJ level and the electrocardiogram waveform in the fourth field 4 and the fifth field 5 in the following manner.

### <Output of STJ Level>

When the electrocardiograph 10 is the derived 18-lead, the electrocardiograph 10 transmits electrocardiogram data including a lead waveform in the case of viewing the heart from 18 points of view, to the control operation unit 150 through the electrocardiograph I/F 120. When the electrocardiogram analysis report is printed on a paper, the control operation unit 150 matches the positions of the point of views and the heart at which the electrocardiograph 10 acquires the electrocardiogram data, to the positions on the paper on which the electrocardiogram analysis report is output, with respect to each of the limb leads and the chest lead. The control operation unit 150 determines the output position of the STJ level at a position spaced apart by a distance corresponding to a size of the STJ level in the direction from the position of the heart to each of the points of view on the paper. The output unit 140 plots information of the STJ level in the shape of a circle graph in association with the position of the point of view, around the position of the heart viewed from the lower surface and the front surface of the human body, based on the output position. That is, the information of the STJ level is arranged and output in association with the direction of the heart.

As illustrated in Fig. 4, with respect to each of the limb leads and the chest lead, assuming that the position of the heart on the paper is H, the output unit 140 draws an arc (referenced as 'Arc') by connecting the points at which the STJ level is zero (reference value) with respect to each lead. In this embodiment, the respective points of the arc are equidistant from the position H of the heart. The output unit 140 plots the information of the STJ level at a position spaced apart by a distance corresponding to a size of the STJ level in the direction from the position H of the heart to each of the points of view on the paper. Specifically, as the STJ level increases, the information of the STJ level is plotted at the more distant position from the position H of the heart. When the STJ level is positive, the information of the STJ level is plotted outside the arc, and when the STJ level is negative, the information of the STJ level is plotted inside the arc. Therefore, the medical staff can easily understand the displacement of the corresponding-lead STJ level from the arc.

Also, when viewed from each of the points of view, if a part of the heart is, for example, limb leads, it is output with characters such as 'side wall' or 'lower wall', and if apart of the heart is a chest lead, it is output with characters such as 'right ventricle', 'front wall', 'side wall', or 'back wall'. Therefore, the medical staff can easily understand the relation between the STJ level and each part of the heart. Also, 'Frontal' represents a frontal plane of the chest, and 'Horizontal' represents a horizontal plane of the chest. The frontal plane of the chest and the horizontal plane of the chest may be represented by using the illustration of the human body instead of the characters.

Also, in this embodiment, a normal range of the STJ level is output with hatching in a band shape along the arc. For example, the normal range of the STJ level is -1 ∼ 1 [mm]. Therefore, the medical staff can easily determine whether the STJ level is a normal value or an abnormal value. That is, in this embodiment, in the electrocardiogram analysis report, the range corresponding to the point of a predetermined value such as the normal range is displayed in accordance with the information of the measurement value.

Also, in this embodiment, the printer plots the information of the STJ level on a paper by using a mark corresponding to the positive/negative sign of the STJ level. In the example illustrated in Fig. 4, when the STJ level is positive (ST increase), a black square sign is used as the mark, and when the STJ level is negative (ST decrease), a square sign is used as the mark. Therefore, the medical staff can plainly determine whether the STJ level is positive or negative. Without being limited to the above-mentioned mark, other characters, signs, or diagrams may be used as the mark if they can be identified from each other. Alternatively, the color of the mark may be changed according to the STJ level, or the mark may be flickered. That is, in the electrocardiogram analysis report, the information of the measurement value may be displayed with a different mark or in a different color according to the magnitude relation between the measurement value and a reference value.

In addition, in this embodiment, since a line graph connecting the marks by a straight line is used, it is easy to visually understand an ST increase and an ST decrease in the relation between a certain part of the heart and other parts of the heart. For example, in the example illustrated in Fig. 4, an ST decrease is found at the front wall of the heart, while an ST increase is found at the rear wall of the heart. Therefore, the medical staff can wholly understand a phenomenon occurring in the heart of the subject.

### <Output of Electrocardiogram Waveform>

As illustrated in Fig. 4, with respect to the limb leads of the fourth field 4, the control operation unit 150 sequentially arranges and outputs the electrocardiogram waveforms in the order of the aVL lead, the I lead, the -aVR lead, the II lead, the aVF lead, and the III lead in the clockwise direction, around the position H of the heart, such that each part of the heart corresponds to the waveform of each lead when viewed from six points of view.

Also, with respect to the chest lead of the fifth field 5, the control operation unit 150 sequentially arranges and outputs the electrocardiogram waveforms on the paper in the order of the syn-V5R lead, the syn-V4R lead, the syn-V3R lead, the V1 lead, the V2 lead, the V3 lead, the V4 lead, the V5 lead, the V6-lead, the syn-V7 lead, the syn-V8 lead, and the syn-V9 lead in the clockwise direction, around the position H of the heart, such that each part of the heart corresponds to the waveform of each lead when viewed from 12 points of view.

In this manner, in this embodiment, since the electrocardiogram waveform is arranged and output in association with the direction of the heart, the relation between the electrocardiogram waveform of each lead and each part of the heart can be easily recognized.

Also, in this embodiment, with respect to the chest lead of the fifth field 5, since the electrocardiogram waveform is output in the direction from the feet to the head with the back directed downward, the arrangement of the electrocardiogram waveform can be matched with the arrangement of the part of a heart in a diagnostic imaging apparatus such as CT or MRI.

Also, in this embodiment, the additional lead such as the right side lead (such as syn-V3R or syn-V4R) or the rear wall lead (such as V7 or V8) is output in addition to the standard 12-lead, thereby making it easy for the medical staff to predict the phenomenon occurring in the heart.

Also, in this embodiment, each electrocardiogram waveform is output together with the value of the STJ level. By outputting electrocardiogram waveform together with the value of the STJ level, the medical staff can immediately understand the level of the STJ level even when the STJ level greatly deviates from the arc. That is, in this embodiment, in the electrocardiogram analysis report, the numerical value of the measurement value is displayed in accordance with the information of the measurement value.

In this manner, in this embodiment, since the STJ level is output in the shape of a circle graph and the value of the STJ level is output, a change in the STJ level can be schematically understood from the circle graph and the actual value of the STJ level can be found out. Therefore, the medical staff can accurately determine how much the STJ level has actually changed.

As above, according to this embodiment, by displaying the electrocardiogram waveform together with the plot of the information of the measurement value, it is possible to output the electrocardiogram analysis report that makes it easy to predict which part of the heart a trouble has occurred. Also, with respect to the chest lead, since the electrocardiogram waveform is output in the same direction as other diagnostic imaging apparatuses, it is possible to output the electrocardiogram analysis report that makes it easy to understand the correspondence relation between the electrocardiogram and the image of the diagnostic imaging apparatus.

Also, in this embodiment, the configuration in which the electrocardiogram analysis apparatus is separated from the electrocardiograph has been described. However, the electrocardiogram analysis apparatus according to this embodiment may be embedded in the electrocardiograph.

Also, in this embodiment, the case where the electrocardiogram analysis report outputs the first field 1 to the fifth field 5 in one-page paper has been described. However, the electrocardiogram analysis report may be output over a plurality of pages.

Also, with respect to the chest lead of the fifth field 5, in order to clarify that the direction is from the feet to the head with the back directed downward, characters or an illustration may be output in the fifth field 5 to indicate that the direction is from the feet to the head with the back directed downward.

### (Other Embodiments)

In addition, the electrocardiogram analysis apparatus may be included in a biological information monitor. In this embodiment, the biological information monitor functions as the electrocardiogram analysis apparatus. The biological information monitor is a medical device for monitoring the body state of a subject by continuously measuring biological information of the subject.

In this embodiment, in a monitor screen of the biological information monitor, a predetermined point of the screen displayed to monitor the biological information of the subject is selected by operating, for example, a touch key, and the electrocardiogram analysis report illustrated in Fig. 4 is popped up on the monitor screen. Also, the screen of the electrocardiogram analysis report may be switched with the monitoring screen.

An electrocardiogram analysis report specific to a biological information monitor, a biological information monitor serving as the electrocardiogram analysis apparatus outputting the electrocardiogram analysis report to the biological information monitor, and an electrocardiogram analysis program will be described in (1) to (4) with reference to Figs. 5 to 7. Also, among the configurations of the biological information monitor according to this embodiment, the same configuration as the configuration of a conventional biological information monitor will not be described herein.

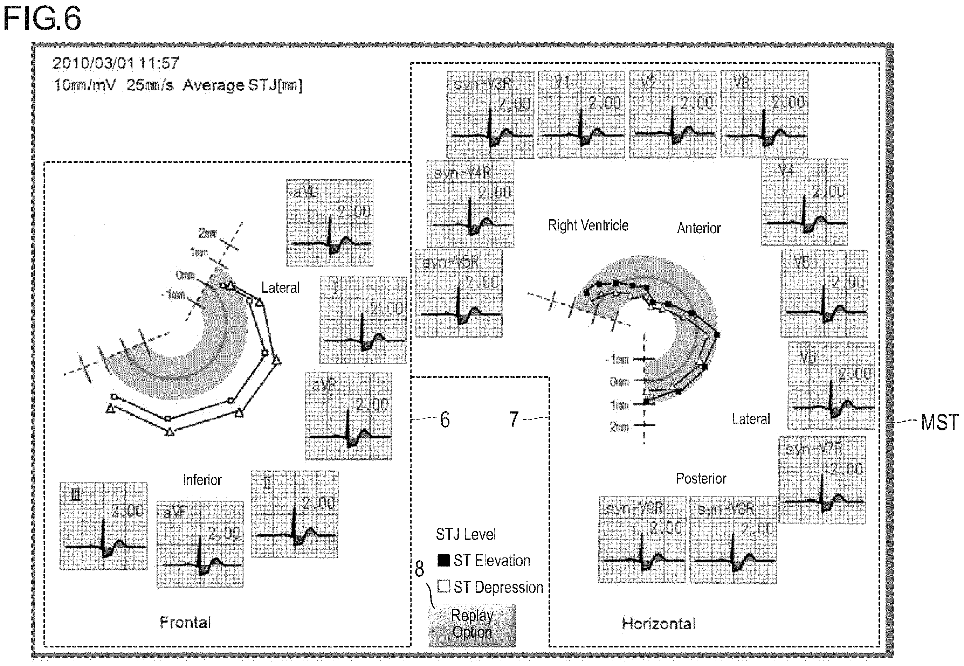

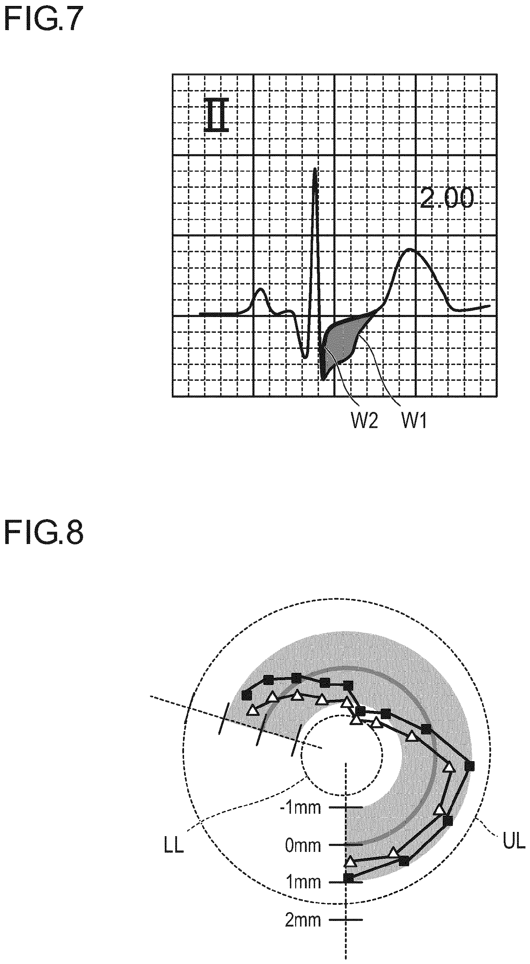

Fig. 5 is a block diagram illustrating a schematic configuration of a biological information monitor according to another embodiment, and Fig. 6 is a diagram illustrating an example of an electrocardiogram analysis report displayed on a biological information monitor according to another embodiment. Fig. 7 is a diagram for describing an electrocardiogram waveform comparison function according to another embodiment, and Fig. 8 is a diagram for describing an alarm range display function according to another embodiment.

As illustrated in Fig. 5, an electrocardiogram analysis monitor 200 according to this embodiment includes a storage unit 210, an input/output interface (hereinafter, referred to as input/output I/F) 220, an input unit 230, an output unit 240, and a control operation unit 250, and these elements are communicatively connected to each other.

Since the storage unit 210, the input unit 230, and the output unit 240 are the same as the storage unit 110, the input unit 130, and the output unit 140 illustrated in Fig. 1, a detailed description will be omitted.

The input/output I/F 220 acquires an electrical signal from a heart though a limb electrode portion 11 and a chest electrode portion 12, converts the electrical data into electrocardiogram data, and transmits the electrocardiogram data to the control operation unit 250.

The control operation unit 250 includes a CPU, and executes various software programs and various operations. In this embodiment, the control operation unit 250 executes an electrocardiogram analysis program for a biological information monitor to acquire electrocardiogram data, analyze an electrocardiogram waveform, and generate image data of an electrocardiogram analysis report. The control operation unit 250 transmits the image data of the electrocardiogram analysis report to the output unit 240, and stores the image data of the electrocardiogram analysis report in the storage unit 210.

In this embodiment, the control operation unit 250 functions as analysis part. Also, the output unit 240 and the control operation unit 250 functions as output part. The following functions (1) to (4) are implemented by executing the electrocardiogram analysis program for the biological information monitor by the control operation unit 250. Also, since other functions of the electrocardiogram analysis program for the biological information monitor are the same as those of the electrocardiogram analysis program for the electrocardiograph described above, a detailed description thereof will be omitted.

### (1) Real-time Update Function

The real-time update function of the biological information monitor is a function of updating the display of an electrocardiogram waveform and an STJ level in real time in accordance with the update of the electrocardiogram data of the monitor. The biological information monitor acquires electrocardiogram data at predetermined intervals and continuously measures an electrocardiogram waveform. Therefore, unlike the electrocardiograph, the biological information monitor can monitor the electrocardiogram waveform and the STJ level in real time. The electrocardiogram data acquired at the predetermined intervals is accumulated in the storage unit 210 embedded in the biological information monitor or is stored in an external storage device (not illustrated) of the biological information monitor.

The electrocardiogram data acquisition interval may be, for example, 0.1 second to several seconds. Also, the electrocardiogram waveform and the STJ level displayed on the monitor may be automatically updated, for example, every 1 to several seconds, preferably, every 2 to 5 seconds. Also, if necessary, the update interval may be changed by the medical staff operating the biological information monitor. Also, the display may be updated at a predetermined time by pressing an update button of the monitor screen by the medical staff.

As illustrated in Fig. 6, the biological information monitor simultaneously displays the electrocardiograph waveform and the STJ level on an ST display screen MST as the electrocardiogram analysis report. For example, the ST display screen MST is pop-up displayed on the monitor screen of the biological information monitor by selecting a predetermined point through a touch key operation by the medical staff. The displayed ST display screen MST is closed by a predetermined touch key operation, so that the monitor screen can return to the state prior to the display of the ST display screen MST.

Also, the electrocardiogram wave illustrated in Fig. 6 is a diagrammatic drawing, and does not necessarily reflect the actual shape of the electrocardiogram waveform.

In the ST display screen MST, a limb leads STJ level and an electrocardiogram waveform are output in a frontal plane display region 6, and a chest lead STJ level and an electrocardiogram waveform are output in a horizontal plane display region 7. Unlike the electrocardiogram analysis report for the electrocardiograph, the ST display screen MST mainly displays information related to the ST. In the example illustrated in Fig. 6, an electrocardiogram waveform and an STJ level corresponding to each part of the heart at the current time are displayed in the frontal plane display region 6 and the horizontal plane display region 7.

In Fig. 6, the STJ level is displayed using a square sign or a black square sign. The meanings of the square signal and the black square sign will be described below. A triangle sign represents a reference STJ level. The reference STJ level is a reference STJ level based on the electrocardiogram data acquired at the previous time point, or is a predetermined STJ level. Also, although Fig. 6 illustrates the case of displaying the current STJ level and the reference STJ level together, the reference STJ level may not be displayed. Also, the signs such as the square sign, the black square sign, and the triangle sign are illustrated for describing this embodiment, and it is needless to say that various other signs may be used. The reference STJ level will be described in detail in the following comparison function (2).

In this manner, since the biological information monitor has the real-time update function, the medical staff can easily refer to the latest electrocardiogram waveform and STJ level.

### (2) Comparison Function

The comparison function of the biological information monitor is a function of simultaneously displaying an electrocardiogram waveform and an STJ level at one or more desired previous time points and an electrocardiogram waveform and an STJ level at a current time point. The biological information monitor displays the electrocardiogram waveform and the STJ level at each time point in the shape that can be easily discriminated (for example, signs, colors, and shading). In the example illustrated in Fig. 6, the STJ level at a desired previous time point is displayed in a triangle sign as the reference STJ level.

As illustrated in Fig. 6, in the frontal plane display region 6, the STJ level at the current time point is smaller in value than the reference STJ level at the desired previous time point. The current STJ level smaller than the reference STJ level is displayed in the square. On the other hand, in the horizontal plane display region 7, the STJ level at the current time point is greater in value than the reference STJ level at the desired previous time point. The current STJ level greater than the reference STJ level is displayed in the black square.

In this manner, when the current STJ level is smaller than the reference STJ level, the current STJ level is displayed in the square, and when the current STJ level is greater than the reference STJ level, the current STJ level is displayed in the black square. Therefore, the medical staff can easily understand the magnitude relation between the current STJ level and the reference STJ level.

Also, the biological information monitor has a function of comparing an electrocardiogram waveform at a previous time point with an electrocardiogram waveform at a current time point. For example, as illustrated in Fig. 7, an electrocardiogram waveform at a previous time point (hereinafter, referred to as a reference electrocardiogram waveform W1) is superimposed on an electrocardiogram waveform W2 at a current time point. In addition, an ST portion changed into the current electrocardiogram waveform W2 from the reference electrocardiogram waveform W1 is painted in a specific color (gray in Fig. 7). Also, the painting color may be a single color or may include a plurality of colors.

In this manner, since the biological information monitor has the comparison function, it is possible to comparatively evaluate how much the STJ level and the electrocardiogram wave have changed from any time point, or whether the STJ level and the electrocardiogram wave have not changed. Also, the superimposition of the electrocardiogram waveforms may use electrocardiogram waveforms at three or more time points.

### (3) Replay Function

The replay function of the biological information monitor is a function of sequentially reproducing the display of the electrocardiogram waveform and the STJ level between two or more desired time points at predetermined intervals. The replayed display may be replaced with the next-replayed display, or may remain. When the replayed display remains, the displays at all the time points can be compared. In this case, the display at the respective time points is displayed in the shape that can be easily discriminated (such as signs, colors, and shading). The biological information monitor reproduces the display of the electrocardiogram waveform and the STJ level by using the electrocardiogram data accumulated in the storage unit 210 or the external storage device (not illustrated).

When a replay option 8 illustrated in Fig. 6 is selected, a setup screen (not illustrated) for setting a replay option is displayed. A replay period, a replay direction, and a replay speed may be set through the setup screen.

The replay period is a period during which the electrocardiogram waveform and the STJ level are displayed, and are designated with a start date and time and an end date and time.

Also, the replay direction is a direction in which the display of the electrocardiogram waveform and the STJ level is to be replayed from the past to the present or from the present to the past, and one of them is selected in execution of the display.

Also, the replay speed is a speed at which the display of the electrocardiogram waveform and the STJ level is replayed. The replay speed may be changed, for example, gradually or continuously. For example, the replay speed may be selected among a first speed, a second speed higher than the first speed, and a third speed higher than the second speed.

### (4) Alarm Range Display Function

The alarm range display function of the biological information monitor is a function of displaying an alarm range of the STJ level. A normal range of the STJ level is displayed with shading in Fig. 4, but it may be changed according to subjects. In this case, for example, a shading display range may be changed, or an upper limit value and a lower limit value of an alarm may be displayed with lines.

The medical staff may set the upper limit value and the lower limit value of the alarm by operating a touch key. When the STJ level deviates from the range from the upper limit value to the lower limit value of the alarm, the biological information monitor informs the medical staff by outputting a warning tone or the like. The upper limit value and the lower limit value of the alarm may be set independently of the normal range of the STJ level.

As illustrated in Fig. 8, the set upper limit value UL and lower limit value LL of the alarm are displayed in the plot of the STJ level on a concentric circle around the position of the heart. In the example illustrated in Fig. 8, the upper limit value UL and the lower limit value LL of the alarm are displayed in a dotted-line circle. Also, the normal range of the STJ level is displayed in gray.

In this manner, since the biological information monitor displays the alarm range, the medical staff can easily determine whether the ST has deviated from the alarm range by any lead.

As above, the electrocardiogram analysis report, the electrocardiogram analysis apparatus, and the electrocardiogram analysis program according to this embodiment has been described. However, needless to say, various modifications can be made by those skilled in the art without departing from the scope of the invention as defined in the claims.

## Claims

1. An electrocardiogram analysis apparatus (100) comprising:
an analysis part (150) configured to analyze an electrocardiogram waveform collected from a subject; and
an output part (140) configured to output an electrocardiogram analysis report based on an analysis result of the electrocardiogram waveform,
wherein the analysis part (150) calculates a measurement value based on each of a limb lead waveform and a chest lead waveform included in the electrocardiogram waveform, and the output part (140) includes:
a frontal plane output unit configured to output the limb lead waveform and information in the shape of a circle graph about the measurement value calculated from the limb lead waveform; and
a horizontal plane output unit configured to output the chest lead waveform and information in the shape of a circle graph about the measurement value calculated from the chest lead waveform;
wherein the measurement value is selected from the group consisting of a wave height or width of a P wave, a Q wave, an R wave, an S wave, a T wave, and a QRS wave, a PR interval, a QT interval, a QTc interval, and an ST level;
**characterized in that** each circle graph comprises an arc connecting the points at which the measurement value is zero with respect to each respective lead, the respective points of the arc being equidistant from a predetermined center point (H) corresponding to the position of the heart;
wherein for each of the limb leads and the chest leads, the frontal plane output unit and the horizontal plane output unit output the information about the measurement value in the respective circle graph at a position on an output surface spaced apart by a distance corresponding to the size of the measurement value in a direction from the predetermined center point (H) to the relevant lead waveform, wherein when the measurement value is positive, the information about the measurement value is plotted outside the arc, and when the measurement value is negative, the information about the measurement value is plotted inside the arc; and
wherein the horizontal plane output unit is configured to output the chest lead waveform and information about the measurement value in an arrangement which corresponds to the position of the corresponding chest electrode when viewed from the lower side of a human body with the back directed downward.

2. An electrocardiogram analysis apparatus (100) according to claim 1, wherein the electrocardiogram waveforms of the chest leads are arranged in the order of the V1 lead, the V2 lead, the V3 lead, the V4 lead, the V5 lead, and the V6 lead in the clockwise direction, around the predetermined center point corresponding to the position of the heart in order to be matched with the arrangement of the part of a heart in an image of a diagnostic imaging apparatus by which an image is created in the direction from the foot side to the heart side with the back directed downward.

3. An electrocardiogram analysis apparatus (100) according to claim 1 or 2, wherein the electrocardiogram waveforms of the limb leads are arranged in the order of the aVL lead, the I lead, the -aVR lead, the II lead, the aVF lead, and the III lead in the clockwise direction, around the predetermined centerpoint (H) corresponding to the position of the heart.

4. The electrocardiogram analysis apparatus (100) according to any preceding claim, wherein the waveform and the measurement value are updated by the latest information prior to output.

5. The electrocardiogram analysis apparatus (100) according to any preceding claim, wherein with respect to the waveform and the measurement value, the information at different times is continuously output.

6. The electrocardiogram analysis apparatus (100) according to any preceding claim, wherein the output part (140) includes at least one of a display and a printer.

7. The electrocardiogram analysis apparatus (100) according to any preceding claim, wherein with respect to the waveform and the measurement value, the electrocardiogram waveforms at different times are output in a superimposed manner, and a changed ST portion is painted in a specific color.

8. The electrocardiogram analysis (100) apparatus according to any preceding claim, wherein an upper limit value and a lower limit value of an alarm about the measurement value are output.

9. A computer-readable recording medium (110) storing an electrocardiogram analysis program causing a computer to perform:
an analysis function (S101) of analyzing an electrocardiogram waveform collected from a subject; and
an output function (S103) of outputting an electrocardiogram analysis report based on an analysis result of the electrocardiogram waveform,
wherein the analysis function calculates a measurement value based on each of a limb lead waveform and a chest lead waveform included in the electrocardiogram waveform, and the output function includes:
a frontal plane output function of outputting the limb lead waveform and information in the shape of a circle graph about the measurement value calculated from the limb lead waveform; and
a horizontal plane output function of outputting the chest lead waveform and information in the shape of a circle graph about the measurement value calculated form the chest lead waveform;
wherein the measurement value is selected from the group consisting of a wave height or width of a P wave, a Q wave, an R wave, an S wave, a T wave, and a QRS wave, a PR interval, a QT interval, a QTc interval, and an ST level;
**characterized in that** each circle graph comprises an arc connecting the points at which the measurement value is zero with respect to each respective lead, the respective points of the arc being equidistant from a predetermined center point (H) corresponding to the position of the heart;
wherein for each of the limb leads and the chest leads the frontal plane output function and the horizontal plane output function display the information about the measurement value in the respective circle graph at a position on a display surface spaced apart by a distance corresponding to a size of the measurement value in a direction from a predetermined center point to the relevant lead waveform, wherein when the measurement value is positive, the information about the measurement value is plotted outside the arc, and when the measurement value is negative, the information about the measurement value is plotted inside the arc; and
wherein the horizontal plane output function outputs the chest lead waveform and information about the measurement value in an arrangement which corresponds to the position of the corresponding chest electrode when viewed from the lower side of a human body with the back directed downward.

10. A computer-readable recording medium according to claim 9, wherein the electrocardiogram waveforms of the chest leads are arranged in the order of the V1 lead, the V2 lead, the V3 lead, the V4 lead, the V5 lead, and the V6 lead in the clockwise direction, around the position of the heart in order to be matched with the arrangement of the part of a heart in an image of a diagnostic imaging apparatus by which an image is created in the direction from the foot side to the heart side with the back directed downward.

11. A computer-readable recording medium according to claim 9 or 10, wherein the electrocardiogram waveforms of the limb leads are arranged in the order of the aVL lead, the I lead, the -aVR lead, the II lead, the aVF lead, and the III lead in the clockwise direction, around the predetermined center point (H) corresponding to the position of the heart.

12. The computer-readable recording medium according to any of claims 9 to 11, wherein the waveform and the measurement value are updated by the latest information prior to output.

13. The computer-readable recording medium according to any of claims 9 to 12, wherein with respect to the waveform and the measurement value, the information at different times is continuously output.

14. The computer-readable recording medium according to any of Claims 9 to 13, wherein with respect to the waveform and the measurement value, the electrocardiogram waveforms at different times are output in a superimposed manner, and a changed ST portion is painted in a specific color.

15. The computer-readable recording medium according to any of Claims 9 to 14, wherein an upper limit value and a lower limit value of an alarm about the measurement value are output.

## Patentansprüche

1. Ein EKG-Analysegerät (100), umfassend:
ein für die Analyse der von einer Versuchsperson erfassten EKG-Wellenform ausgelegtes Analyseteil (150); und
ein für die Ausgabe eines EKG-Analyseberichts anhand eines Analyseergebnisses der EKG-Wellenform ausgelegtes Ausgabesteil (140), wobei der Analyseteil (150) einen Messwert anhand der Wellenform aller Extremitäten berechnet
und eine in der EKG-Wellenform enthaltene Brustableitung-Wellenform, und das Ausgangsteil (140) umfasst:
eine Frontalebenen-Ausgabeeinheit, die für die Ausgabe der Wellenform der Extremitätenableitung und für die Ausgabe von Informationen über den Messwert in Form eines Kreisdiagramms ausgelegt ist, wobei der Messwert aus der Wellenform der Extremitätenableitung berechnet wird; und
eine Horizontalebenen-Ausgabeeinheit, die für die Ausgabe der Wellenform der Brustableitung und für die Ausgabe von Informationen über den Messwert in Form eines Kreisdiagramms ausgelegt ist, wobei der Messwert aus der Wellenform der Brustableitung berechnet wird; und
wobei der Messwert anhand der Wellenhöhe oder -breite einer P-Welle, einer Q-Welle, einer R-Welle, einer S-Welle, einer T-Welle und einer QRS-Welle, eines PR-Intervalls, eines QT-Intervalls, eines QTc-Intervalls und einer ST-Stecke definiert wird;
**dadurch gekennzeichnet, dass** jedes Kreisdiagramm einen die Punkte verbindenden Kreisbogen aufweist, an denen der Messwert in Bezug auf die jeweiligen Ableitungen null beträgt, wobei die jeweiligen Kreisbogenpunkte von einem vorbestimmten Mittelpunkt (H) entsprechend der Position des Herzens gleich weit voneinander entfernt sind;
wobei die Frontalebenen- und die Horizontalebenen-Ausgabeeinheit die Messwert-Informationen für die Extremitätenableitung und die Brustableitung auf einer Ausgabefläche im jeweiligen Kreisdiagramm an einem solchen Punkt ausgeben, dass deren Abstand der Größe des Messwertes vom vorgegebenen Mittelpunkt (H) zur relevanten Wellenform einer Ableitung entspricht, wobei die Informationen über den Messwert bei einem positiven Messwert außerhalb des Kreisbogens und die Informationen über den Messwert bei einem negativen Messwert innerhalb des Kreisbogens aufgetragen werden;
wobei die Horizontalebenen-Ausgabeeinheit dafür ausgelegt ist, die Wellenform der Brustableitung sowie Informationen über den Messwert so angeordnet auszugeben, dass deren Position der entsprechenden Brust-Elektrode entspricht, wenn ein menschlicher Körper mit dem Rücken nach unten gerichtet von unten aus betrachtet wird.

2. Ein EKG-Analysegerät (100) gemäß Anspruch 1, wobei die EKG-Wellenformen der Brustableitungen im Uhrzeigersinn um den vorgegebenen Mittelpunkt entsprechend der Position des Herzens in der Reihenfolge V1-Ableitung, V2-Ableitung, V3-Ableitung, V4-Ableitung, V5-Ableitung und V6-Ableitung angeordnet sind, damit diese in einem Bild eines diagnostischen Abbildungsgerätes über einen Teil des Herzens gelegt werden können, welches vom Fuß aus in Richtung Herz mit dem Rücken nach unten erzeugt wurde.

3. Ein EKG-Analysegerät (100) gemäß Anspruch 1 oder 2, wobei die EKG-Wellenformen der Extremitätenableitungen im Uhrzeigersinn um den vorgegebenen Mittelpunkt (H) entsprechend der Position des Herzens in der Reihenfolge aVL-Ableitung, I-Ableitung, -aVR-Ableitung, II-Ableitung, aVF-Ableitung und II-Ableitung angeordnet sind.

4. Ein EKG-Analysegerät (100) gemäß einem der vorhergehenden Ansprüche, wobei Wellenform und Messwert vor einer Ausgabe durch die neuesten Informationen aktualisiert werden.

5. Ein EKG-Analysegerät (100) gemäß einem der vorhergehenden Ansprüche, wobei die Informationen in Bezug auf Wellenform und Messwert zu verschiedenen Zeitpunkten kontinuierlich ausgegeben werden.

6. Ein EKG-Analysegerät (100) gemäß einem der vorhergehenden Ansprüche, wobei das Ausgabesteil (140) mindestens einen Bildschirm und einen Drucker aufweist.

7. Ein EKG-Analysegerät (100) gemäß einem der vorhergehenden Ansprüche, wobei die EKG-Wellenformen in Bezug auf Wellenform und Messwert zu verschiedenen Zeitpunkten überlagert ausgegeben werden und eine veränderte ST-Strecke in einer bestimmten Farbe dargestellt wird.

8. Ein EKG-Analysegerät (100) gemäß einem der vorhergehenden Ansprüche, wobei der obere Grenzwert und der untere Grenzwert eines Messwert-Alarms ausgegeben werden.

9. Ein computerlesbares Aufzeichnungsmedium (110), das ein EKG-Analyseprogramm speichert und bewirkt, dass ein Computer folgende Funktionen ausführt:
die Analyse (S101) der von einer Versuchsperson erfassten EKG-Wellenform; und
die Ausgabe (S103) eines EKG-Analyseberichts anhand eines Analyseergebnisses der EKG-Wellenform
wobei die Analysefunktion einen Messwert anhand der Wellenformen aller Extremitäten und der in der EKG-Wellenform enthaltenen Brustableitung-Wellenform berechnet, und die Ausgabefunktion umfasst:
eine Frontalebenen-Ausgabefunktion für die Ausgabe der Extremitätenableitung-Wellenform und der Informationen über den Messwert in Form eines Kreisdiagramms, wobei der Messwert aus der Extremitätenableitung-Wellenform berechnet wird; und
eine Horizontalebenen-Ausgabefunktion für die Ausgabe der Wellenform der Brustableitung und der Informationen über den Messwert in Form eines Kreisdiagramms, wobei der Messwert aus der Wellenform der Brustableitung berechnet wird;
wobei der Messwert anhand der Wellenhöhe oder -breite einer P-Welle, einer Q-Welle, einer R-Welle, einer S-Welle, einer T-Welle und einer QRS-Welle, eines PR-Intervalls, eines QT-Intervalls, eines QTc-Intervalls und einer ST-Stecke definiert wird;
**dadurch gekennzeichnet, dass** jedes Kreisdiagramm einen die Punkte verbindenden Kreisbogen aufweist, an denen der Messwert in Bezug auf die jeweiligen Ableitungen null beträgt, wobei die jeweiligen Kreisbogenpunkte von einem vorbestimmten Mittelpunkt (H) analog zur Position des Herzens gleich weit voneinander entfernt sind;
wobei die Frontalebenen- und die Horizontalebenen-Ausgabefunktion die Messwert-Informationen für die Extremitätenableitungen und die Brustableitung auf einer Ausgabefläche im jeweiligen Kreisdiagramm an einem solchen Punkt anzeigen, dass deren Abstand der Größe des Messwertes vom vorgegebenen Mittelpunkt zur relevanten Wellenform einer Ableitung entspricht, wobei die Informationen über den Messwert bei einem positiven Messwert außerhalb des Kreisbogens und die Informationen über den Messwert bei einem negativen Messwert innerhalb des Kreisbogens aufgetragen werden;
wobei die Horizontalebenen-Ausgabefunktion dafür ausgelegt ist, die Wellenform der Brustableitung sowie Informationen über den Messwert so angeordnet auszugeben, dass deren Position der entsprechenden Brust-Elektrode entspricht, wenn ein menschlicher Körper mit dem Rücken nach unten gerichtet von unten aus betrachtet wird.

10. Ein computerlesbares Aufzeichnungsmedium gemäß Anspruch 9, wobei die EKG-Wellenformen der Brustableitungen im Uhrzeigersinn um die Position des Herzens in der Reihenfolge V1-Ableitung, V2-Ableitung, V3-Ableitung, V4-Ableitung, V5-Ableitung und V6-Ableitung angeordnet sind, damit diese in einem Bild eines diagnostischen Abbildungsgerätes über einen Teil des Herzens gelegt werden können, welches vom Fuß aus in Richtung Herz mit dem Rücken nach unten erzeugt wurde.

11. Ein computerlesbares Aufzeichnungsmedium gemäß den Ansprüchen 9 oder 10, wobei die EKG-Wellenformen der Extremitätenableitungen im Uhrzeigersinn um den vorgegebenen Mittelpunkt (H) entsprechend der Position des Herzens in der Reihenfolge aVL-Ableitung, I-Ableitung, -aVR-Ableitung, II-Ableitung, aVF-Ableitung und II-Ableitung angeordnet sind.

12. Das computerlesbare Aufzeichnungsmedium gemäß einem der Ansprüche 9 bis 11, wobei Wellenform und Messwert vor einer Ausgabe durch die neuesten Informationen aktualisiert werden.

13. Das computerlesbare Aufzeichnungsmedium gemäß einem der Ansprüche 9 bis 12, wobei die Informationen in Bezug auf Wellenform und Messwert zu verschiedenen Zeitpunkten kontinuierlich ausgegeben werden.

14. Das computerlesbare Aufzeichnungsmedium gemäß einem der Ansprüche 9 bis 13, wobei die EKG-Wellenformen in Bezug auf Wellenform und Messwert zu verschiedenen Zeitpunkten überlagert ausgegeben werden und eine veränderte ST-Strecke in einer bestimmten Farbe dargestellt wird.

15. Das computerlesbare Aufzeichnungsmedium gemäß einem der Ansprüche 9 bis 14, wobei der obere Grenzwert und der untere Grenzwert eines Messwert-Alarms ausgegeben werden.

## Revendications

1. Un appareil d'analyse électrocardiogramme (100) comprenant :
un élément d'analyse (150) configuré pour analyser une courbe électrocardiogramme prise d'un sujet ; et
un élément de sortie (140) configuré pour produire un rapport d'analyse électrocardiogramme basé sur le résultat d'une analyse d'une courbe électrocardiogramme,
dans lequel l'élément d'analyse (150) calcule une valeur de mesure basée sur chaque courbe de dérivation d'un membre
et une courbe de dérivation de la poitrine incluse dans la courbe électrocardiogramme, et l'élément de sortie (140) inclut :
une unité de sortie à plan frontal configurée pour produire la courbe de dérivation du membre et les informations sous forme
d'un graphique rond sur la valeur de mesure calculée depuis la courbe de dérivation du membre ; et une unité de sortie à plan horizontal configurée pour produire la courbe de dérivation de la poitrine et les informations sous forme d'un graphique rond à partir de la courbe de dérivation de la poitrine ;
dans lequel la valeur de mesure est sélectionnée à partir du groupe consistant en une hauteur de vague ou une largeur de vague P, une vague Q, une vague R, une vague S, une vague T, et une vague QRS, un intervalle PR, un intervalle QT, un intervalle QTc, et un niveau ST ;
**caractérisé en ce que** chaque graphique rond comprend un arc connectant les points auxquels la valeur de mesure est zéro par rapport à chaque dérivation respective, les points respectifs de l'arc étant équidistants depuis un point central prédéterminé (H) correspondant à la position du coeur ;
dans lequel pour chacune des dérivations du membre et chacune des dérivations de la poitrine, l'unité de sortie à plan frontal et l'unité de sortie à plan horizontal produisent les informations sur la valeur de mesure dans le graphique rond respectif à une position sur une surface de sortie espacée par une distance correspondant à la taille de la valeur de mesure dans une direction, depuis le point central prédéterminé (H) à la courbe de dérivation adéquate, où lorsque la valeur de mesure est positive, les informations sur la valeur de mesure sont représentées à l'extérieur de l'arc, et lorsque la valeur de mesure est négative, les informations sur la valeur de mesure sont représentées à l'intérieur de l'arc ;
et
dans lequel l'unité de sortie à plan horizontal est configurée pour produire la courbe de dérivation de la poitrine et les informations sur la valeur de mesure dans un arrangement correspondant à la position de l'électrode de la poitrine lorsque visualisée depuis la partie inférieure du corps humain avec le dos orienté vers le bas.

2. Un appareil d'analyse électrocardiogramme (100) selon la revendication 1, dans lequel les courbes de l'électrocardiogramme des dérivations de la poitrine sont arrangées dans l'ordre de la dérivation V1, la dérivation V2, la dérivation V3, la dérivation V4, la dérivation V5, et la dérivation V6 dans le sens des aiguilles d'une montre, autour du point central prédéterminé correspondant à la position du coeur pour s'associer à l'arrangement de la partie d'un coeur dans l'image d'un appareil d'imagerie diagnostique permettant de créer une image dans la direction, depuis le côté du pied au côté du coeur avec le dos orienté vers le bas.

3. Un appareil d'analyse électrocardiogramme (100) selon la revendication 1 ou 2, dans lequel les courbes de l'électrocardiogramme des dérivations du membre sont arrangées dans l'ordre de la dérivation aVL, la dérivation I, la dérivation - aVR, la dérivation II, la dérivation aVF, et la dérivation III dans le sens des aiguilles d'une montre, autour du point central prédéterminé (H) correspondant à la position du coeur.

4. L'appareil d'analyse électrocardiogramme (100) selon toute revendication précédente, dans lequel la courbe et la valeur de mesure sont mises à jour par les dernières informations avant la production.

5. L'appareil d'analyse électrocardiogramme (100) selon toute revendication précédente, dans lequel par rapport à la courbe et à la valeur de mesure, les informations à différents moments sont produites en continu.

6. L'appareil d'analyse électrocardiogramme (100) selon toute revendication précédente, dans lequel l'élément de sortie (140) inclut au moins un des affichages et une imprimante.

7. L'appareil d'analyse électrocardiogramme (100) selon toute revendication précédente, dans lequel par rapport à la courbe et à la valeur de mesure, les courbes de l'électrocardiogramme à différents moments sont produites de manière superposée, et une portion ST modifiée est peinte dans une couleur spécifique.

8. L'appareil d'analyse électrocardiogramme (100) selon toute revendication précédente, dans lequel une valeur limite supérieure et une valeur limite inférieure d'une alarme sur la valeur de mesure sont produites.

9. Un support d'enregistrement lisible par ordinateur (110) qui stocke un programme d'analyse électrocardiogramme permettant à l'ordinateur de réaliser :
une fonction d'analyse (S101) pour analyser une courbe d'électrocardiogramme prise d'un sujet ; et
une fonction de sortie (S103) pour produire un rapport d'analyse électrocardiogramme basé sur un résultat d'analyse de la courbe de l'électrocardiogramme, dans lequel la fonction d'analyse calcule une valeur de mesure basée sur chaque courbe de dérivation d'un membre et une courbe de dérivation de la poitrine incluse dans la courbe de l'électrocardiogramme, et la fonction de sortie inclut :
une fonction de sortie à plan frontal pour produire la courbe de dérivation du membre et les informations sous forme d'un graphique rond sur la valeur de mesure calculée à partir de la courbe de dérivation du membre ; et
une fonction de sortie à plan horizontal pour produire la courbe de dérivation de la poitrine et les informations sous la forme d'un graphique rond sur la valeur de mesure calculée à partir de la courbe de dérivation de la poitrine ;
dans lequel la valeur de la mesure est sélectionnée à partir d'un groupe consistant en une hauteur de vague ou une largeur de vague P, une vague Q, une vague R, une vague S, une vague T, et une vague QRS, un intervalle PR, un intervalle QT, un intervalle QTc et un niveau ST ;
**caractérisé en ce que** chaque graphique rond comprend un arc connectant les points auxquels la valeur de mesure est zéro par rapport à chaque dérivation respective, les points respectifs de l'arc étant équidistants depuis un point central prédéterminé (H) correspondant à la position du coeur ;
dans lequel pour chaque dérivation du membre et chaque dérivation de la poitrine, la fonction de sortie à plan frontal et la fonction de sortie à plan horizontal affichent les informations sur la valeur de mesure dans le graphique rond respectif à une position sur la surface d'affichage espacée par une distance correspondant à une taille de la valeur de mesure dans une direction depuis un point central prédéterminé à la courbe de dérivation adéquate, dans lequel lorsque la valeur de mesure est positive, les informations sur la valeur de mesure sont représentées à l'extérieur de l'arc, et lorsque la valeur de mesure est négative, les informations sur la valeur de mesure sont représentées à l'intérieur de l'arc ; et
dans lequel la fonction de sortie à plan horizontal produit la courbe de dérivation de la poitrine et les informations sur la valeur de mesure dans un arrangement qui correspond à la position de l'électrode de la poitrine correspondant lorsque visualisé depuis la partie inférieure du corps humain avec le dos orienté vers le bas.

10. Un support d'enregistrement lisible par ordinateur selon la revendication 9, dans lequel les courbes de l'électrocardiogramme des dérivations de la poitrine sont arrangées dans l'ordre de la dérivation V1, la dérivation V2, la dérivation V3, la dérivation V4, la dérivation V5, et la dérivation V6 dans le sens des aiguilles d'une montre, autour de la position du coeur pour s'associer à l'arrangement de la partie du coeur dans une image d'un appareil d'imagerie diagnostique qui permet de créer une image dans la direction depuis le côté du pied au côté du coeur avec le dos orienté vers le bas.

11. Un support d'enregistrement lisible par ordinateur selon la revendication 9 ou 10, dans lequel les courbes de l'électrocardiogramme des dérivations du membre sont arrangées dans l'ordre de la dérivation aVL, la dérivation I, la dérivation -aVR, la dérivation II, la dérivation aVF, et la dérivation III dans le sens des aiguilles d'une montre, autour du point central prédéterminé correspondant à la position du coeur.

12. Le support d'enregistrement lisible par ordinateur selon les revendications 9 à 11, dans lequel la courbe et la valeur de mesure sont mises à jour par les dernières informations avant la production.

13. Le support d'enregistrement lisible par ordinateur selon les revendications 9 à 12, dans lequel par rapport à la courbe et à la valeur de mesure, les informations à différents moments sont produites en continu.

14. Le support d'enregistrement lisible par ordinateur selon les revendications 9 à 13, dans lequel par rapport à la courbe et à la valeur de mesure, les courbes de l'électrocardiogramme à différents moments sont produites de manière superposée, et une portion ST modifiée est peinte dans une couleur spécifique.

15. Le support d'enregistrement lisible par ordinateur selon les revendications 9 à 14, dans lequel une valeur limite supérieure et une valeur limite inférieure d'une alarme sur la valeur de mesure sont produites.
